# EUROPÄISCHE PATENTSCHRIFT

(11) **EP 3 285 659 B1**
(45) Veröffentlichungstag und Bekanntmachung des Hinweises auf die Patenterteilung: **22.09.2021**
(21) Anmeldenummer: 16706162.1
(22) Anmeldetag: 19.02.2016
(51) Int. Cl.: A61B 17/12, A61B 17/00

(54) **MEDIZINISCHES IMPLANTAT ZUM VERSCHLUSS EINER DEFEKTÖFFNUNG, EINES GEFÄSSES, EINES ORGANWEGS ODER EINER ANDEREN ÖFFNUNG IN EINEM MENSCHLICHEN ODER TIERISCHEN KÖRPER**
MEDICAL IMPLANT FOR CLOSURE OF A DEFECT APERTURE, A VESSEL, AN ORGAN PATH OR ANOTHER APERTURE IN A HUMAN OR ANIMAL BODY
IMPLANT MÉDICAL POUR LA FERMETURE D'UNE OUVERTURE DÉFECTUEUSE, D'UN VAISSEAU, D'UN CONDUIT D'ORGANE OU D'UNE AUTRE OUVERTURE DANS UN CORPS HUMAIN OU ANIMAL

(30) Priorität: 24.04.2015 DE 202015102060 U
(43) Veröffentlichungstag der Anmeldung: 28.02.2018
(73) Patentinhaber: PFM Medical AG, 50996 Köln (DE)
(72) Erfinder: SCHÄFER, Joachim, 66620 Nonnweiler (DE)
(74) Vertreter: Hohendorf, Carsten
(86) Internationale Anmeldenummer: PCT/EP2016/053547
(87) Internationale Veröffentlichungsnummer: WO 2016/169671

(56) Entgegenhaltungen:
- WO-A1-01/49185
- WO-A2-2004/064671
- DE-A1-102007 038 446
- US-A1- 2005 187 564

## Beschreibung

Die Erfindung betrifft ein medizinisches Implantat zum Verschluss einer Defektöffnung, eines Gefäßes, eines Organwegs oder einer anderen Öffnung in einem menschlichen oder tierischen Körper. Das erfindungsgemäße medizinische Implantat umfasst einen Grundkörper und wenigstens eine Faser.

Medizinische Implantate zum Verschluss von Defektöffnungen, Gefäßen, Organwegen oder anderen Öffnungen in einem menschlichen oder tierischen Körper sind in den verschiedensten Formen bekannt. Derartige medizinische Implantate werden beispielsweise an einem gewünschten Ort mittels eines Ballon-Katheters expandiert oder bestehen aus einem selbstexpandierenden Material wie z.B. einem Formgedächtnismaterial. Vorzugsweise wird das medizinische Implantat mittels eines minimalinvasiven Verfahrens über einen Katheter in den menschlichen oder tierischen Körper implantiert.

Während des Transports des medizinischen Implantats durch den Katherter an den gewünschten Ort in dem menschlichen oder tierischen Körper weist das medizinische Implantat eine Primärform auf, welche im Wesentlichen langgestreckt ist. Das medizinische Implantat hat also in der Primärform ein großes Verhältnis von Längsausdehnung zu Querausdehnung. Beim Verlassen des Katheters nimmt das medizinische Implantat eine Sekundärform an, um die Defektöffnung, das Gefäß, den Organweg oder die andere Öffnung des menschlichen oder tierischen Körpers zu verschließen. Aus dem Stand der Technik sind eine Vielzahl von Sekundärformen bekannt, um den Organweg oder die andere Öffnung des menschlichen oder tierischen Körpers zu verschließen. Beispielsweise ist der Grundkörper eines medizinischen Implantats in der Sekundärform derart aufgewickelt ausgebildet, dass der Grundkörper eine Trichterform aufweist.

Ferner ist es bekannt thrombogene Fasern an dem medizinischen Implantat anzubringen, um eine Embolisierwirkung zu verbessern.

Hierzu offenbart beispielsweise die EP 0 750 480 B1, dass thrombogene Fasern aus handelsüblichem Z-Twist-Dacron-Fasermaterial in regelmäßigen Abständen über die Länge der Windungen einer Spirale zwischen eng benachbarten Windungen von dieser angeordnet werden. Diese thrombogenen Fasern ragen dann radial zwischen den Windungen von der Primärspirale aus dieser hervor. Ein ähnlicher Aufbau ist auch aus der JP-8131553 bekannt. Ebenfalls sieht die JP-2001079011 einen ähnlichen Aufbau vor.

Gemäß der DE 698 31 889 T2 wird ein medizinisches Implantat offenbart, bei der ein federnder spulenförmig aufgewickelter Draht auf seiner Außenoberfläche mit Schnitten versehen ist, die einerseits der Verbesserung der Flexibilität des Spulendrahts dienen und andererseits dem Anbringen von thrombogenen Fasern. Die Fasern können in den Draht geknotet, mittels eines Klebers dort befestigt, verschmolzen oder mittels eines anderen Verbindungsverfahrens angebracht werden.

Gemäß der DE 698 26 275 T2 wird ein medizinisches Implantat vorgeschlagen, welches eine Primärspirale aufweist, die in diverse Sekundärformen umgeformt werden kann. Entlang der Primärspirale sind thrombogene Fasern eingeflochten. Diese sind jeweils an einem Ende an einer Windung befestigt und durch einige der dazwischen liegenden Windungen hindurchgefädelt, so dass auf der Außenseite der Primärspirale Schlaufen der thrombogenen Fasern abstehen. Alternativ wird offenbart, eine geflochtene Umhüllung aus einem faserigen Material vorzusehen, dass die Primärspirale umgibt. Einen ähnlichen Aufbau offenbart auch die DE 698 33 699 T2, bei der ebenfalls thrombogene Fasern durch die Wendel einer Primärspirale bzw. einer Sekundärspirale gefädelt sind. Hierbei stehen dann ebenfalls Schlaufen thrombogener Fasern aus der Spirale hervor. Einen entsprechenden Aufbau zeigt auch die Gefäßverschlusswendel gemäß der DE 698 26 275 T2.

Aus dem Umfang der Wendel bzw. Spirale herausragende Fasern sind auch aus der US 6,187,027 B1, der EP 1 584 298 A1, der JP-2005237952, der JP-8131553 der JP-2001079011 und der EP102007038446 A1 bekannt.

Eine weitere alternative Lösung zum Anbringen von thrombogenen Fasern an einer Okklusionsspirale ist in der EP 0 778 005 A1 bzw. JP-9276280 offenbart. Hierbei wird eine Vielzahl von Strängen thrombogener Fasern innen durch die Wendel der Okklusionsspirale hindurchgeführt. Die Enden der thrombogenen Faserstränge sind dabei miteinander verbunden.

Ferner offenbart die DE 10 2007 038 446 A1 ein medizinisches Implantat, bei welchem um den spiralförmigen Grundkörper herum thrombogene Fasern gewickelt sind.

Ein weiteres medizinisches Implantat zum Verschluss einer Defektöffnung, eines Gefäßes, eines Organwegs oder einer anderen Öffnung in einem menschlichen oder tierischen Körper wird von der Firma pfm medical ag unter der Bezeichnung "Nit-Occlud VSD" vertrieben. Dieses medizinische Implantat umfasst einen Grundkörper und eine Vielzahl von thrombogenen Fasern. Der Grundkörper ist gegen elastische Rückstellkräfte reversibel von einer Sekundärform in eine Primärform überführbar, wobei der Grundkörper in der Primärform eine langgestreckte Form mit einem großen Verhältnis von Längsausdehnung zu Querausdehnung aufweist und in der Sekundärform eine zumindest teilweise aufgewickelte Form aufweist mit einem kleineren Verhältnis von Längsausdehnung zu Querausdehnung als in der Primärform. Die Nit-Occlud Lê VSD-Spirale ist ein dauerhaftes Implantat zum Verschluss von Ventrikelseptumdefekten (VSD), welches mittels minimal-invasiver Kathetertechnik in die Herzkammer geführt wird. Die Spirale besteht aus Nitinol, einem Material mit Formgedächtnis, und hat im entspannten Zustand (Sekundärform) die Form eines Konus. Um eine beschleunigte Thrombogenität und somit auch kürzere Verschlusszeit zu erzielen, sind im distalen Bereich der Nit-Occlud Lê VSD-Spirale, also in dem Bereich des Konus mit dem größeren Durchmesser, thrombogene Fasern vorgesehen. Dabei sind eine Vielzahl von kurzen thrombogenen Fasern an den Grundkörper geknüpft. Die einzelnen thrombogenen Fasern bestehen dabei aus eine Mehrzahl von einzelnen Filamenten, welche im Mittelbereich der thrombogenen Fasern miteinander verknüpft oder verschränkt sind und an den Enden der thrombogenen Fasern frei beweglich sind. Beispielsweise sind die thrombogenen Fasern bei der Nit-Occlud Lê VSD-Spirale ca. 1 cm lang. Je nach Größe der VSD-Spirale sind ungefähr 10 bis 150 thrombogene Fasern vorgesehen, wobei jede thrombogene Faser beispielsweise 34 oder 36 einzelne Filamente aufweist. Es hat sich jedoch herausgestellt, dass sich die thrombogenen Fasern in einem nassen Zustand derart an die VSD-Spirale anlegen können, dass im spitzzulaufenden Bereich des Konus, also im engen Trichterbereich, eine Öffnung verbleibt. Dadurch verlängert sich die Verschlusszeit.

Es ist daher Aufgabe der vorliegenden Erfindung die aus dem Stand der Technik bekannten medizinischen Implantate zum Verschluss einer Defektöffnung, eines Gefäßes, eines Organwegs oder einer anderen Öffnung in einem menschlichen oder tierischen Körper mit einem Grundkörper und Fasern hinsichtlich der Verschlusszeit zu verbessern.

Die Aufgabe wird erfindungsgemäß gelöst durch ein medizinische Implantat zum Verschluss einer Defektöffnung, eines Gefäßes, eines Organwegs oder einer anderen Öffnung in einem menschlichen oder tierischen Körper, mit einem Grundkörper und wenigstens einer Faser, wobei der Grundkörper gegen elastische Materialkräfte reversibel von einer Sekundärform in eine Primärform überführbar ist, wobei der Grundkörper in der Primärform eine langgestreckte Form aufweist und in der Sekundärform zumindest teilweise aufgewickelt ist und eine Konusform aufweist, welches sich dadurch auszeichnet, dass die wenigstens eine Faser derart mit dem Grundkörper verbunden ist, dass sich die Faser in der Sekundärform des Grundkörpers wenigstens einmal, vorzugsweise mehrmals, quer durch die Konusform erstreckt.

Der Erfindung liegt die Erkenntnis zugrunde, dass sich die wenigstens eine Faser des medizinischen Implantats im nassen Zustand nicht derart an die Innenwandung der Konusform anlegen kann, dass in dem spitzzulaufenden Bereich der Konusform eine Öffnung verbleibt, wenn sich die wenigstens eine Faser quer durch die Konusform erstreckt.

In der Primärform weist der Grundkörper eine langgestreckte Form auf, damit er mittels einer minimal-invasiven Kathetertechnik in den menschlichen oder tierischen Körper implantierbar ist. In dieser Primärform weist der Grundkörper ein großes Verhältnis von Längsausdehnung zu Querausdehnung auf.

Beim Verlassen des zur Implantation verwendeten Katheters nimmt der Grundkörper vorzugsweise selbständig die Sekundärform an, in welcher das aus dem Grundkörper geformte medizinische Implantat die Defektöffnung, das Gefäß, den Organweg oder die andere Körperöffnung in dem menschlichen oder tierischen Körper verschließt. In dieser Sekundärform weist der Grundkörper ein kleineres Verhältnis von Längsausdehnung zu Querausdehnung auf als in der Primärform.

Eine Konusform im Sinne der Erfindung umfasst einen erweiterten Bereich und einen spitzzulaufenden Bereich. Die Konusform entspricht somit einer Trichterform. Vorzugsweise ist der erweiterte Bereich der Konusform am distalen Ende des medizinischen Implantats angeordnet und der spitzzulaufende Bereich der Konusform ist am proximalen Ende des medizinischen Implantats angeordnet. Distal im Sinne der Erfindung ist der der Körpermitte zugewandte Bereich und proximal im Sinne der Erfindung ist der der Körpermitte abgewandte Bereich des medizinischen Implantats.

Ferner hat die erfindungsgemäße Ausgestaltung des medizinischen Implantats ein verringertes Hämolyserisiko, da die Anzahl der verwendeten Fasern deutlich reduziert wurde. Die losen Enden der Fasern können zu einer Zerstörung von Erythrozyten beitragen, was ein maßgebender Faktor für eine Hämolyse ist.

Eine Faser im Sinne der Erfindung ist ein feines, dünnes, fadenähnliches Gebilde, das aus einem pflanzlichen oder tierischen Rohstoff besteht oder synthetisch erzeugt ist.

Nach einer bevorzugten Variante der Erfindung erstreckt sich die wenigstens eine Faser wenigstens einmal durch den Mittelpunkt der Konusform. Wird das medizinische Implantat in den menschlichen oder tierischen Körper implantiert, insbesondere in ein Gefäß oder Organ, wobei das medizinische Implantat in Verbindung mit dem Blutkreislauf steht, kommt es im Bereich des spitzzulaufenden Bereichs zu erhöhten Strömungsgeschwindigkeiten. Damit die wenigstens eine Faser aufgrund der erhöhten Strömungsgeschwindigkeiten nicht derart ihre Lage verändert, dass in dem spitzzulaufenden Bereich eine Öffnung verbleibt, erstreckt sich die wenigstens eine Faser wenigstens einmal durch den Mittelpunkt der Konusform.

In der Erfindung bildet die wenigstens eine Faser in der Konusform eine netzartige Struktur. Eine derartige netzartige Struktur führt zu einer erhöhten Thrombogenität und folglich zu einer verringerten Verschlusszeit. Weiterhin hat eine netzartige Struktur den Vorteil, dass sich die einzelnen Streben der netzartigen Struktur gegenseitig abstützen, so dass die zuvor genannten erhöhten Strömungsgeschwindigkeiten nahezu keine Veränderungen an der netzartigen Struktur verursachen.

Gemäß einer weiteren vorteilhaften Variante der Erfindung ist die wenigstens eine Faser an wenigstens zwei Punkten mit dem Grundkörper verbunden. Eine derartige Ausgestaltung führt auf einfache Art und Weise dazu, dass sich die wenigstens eine Faser in der Sekundärform des Grundkörpers wenigstens einmal quer durch die Konusform erstreckt. Ferner verläuft die wenigstens eine Faser in der Primärform des Grundkörpers nahezu parallel zu dem Grundkörper, so dass die wenigstens eine Faser nahezu keinen Einfluss auf den Durchmesser des medizinischen Implantats in der Primärform hat. Der Durchmesser des medizinischen Implantats in der Primärform ist insbesondere für die minimalinvasive Implantation von Bedeutung, da der Durchmesser des medizinischen Implantats die Größe des zu verwendenden Katheters maßgebend beeinflusst.

In einer zweckmäßigen Variante ist die wenigstens eine Faser an mehr als zwei Punkten mit dem Grundkörper verbunden. Somit ist die wenigstens eine Faser in der Primärform des Grundkörpers wellenförmig an oder um den Grundkörper angeordnet und in der Sekundärform erstrecken sich jeweils die Bereiche der wenigstens einen Faser die nicht mit dem Grundkörper verbunden sind quer durch die Konusform.

Der Abstand zwischen den wenigstens zwei Punkten an welchen die thrombogene Faser mit dem Grundkörper verbunden ist beträgt vorzugsweise zwischen 0,1 und 2,0 cm und insbesondere zwischen 1,0 und 1,5 cm.

Nach einer vorteilhaften Variante der Erfindung steht die wenigstens eine Faser in der Primärform des Grundkörpers maximal 0,5 cm vor dem Grundkörper ab. Dies ist insbesondere für eine Implantation des erfindungsgemäßen Implantats mittels einer minimalinvasiven Kathetertechnik vorteilhaft.

Nach einer besonders zweckmäßigen Variante der Erfindung sind wenigstens zwei Fasern jeweils an wenigstens zwei Punkten mit dem Grundkörper verbunden. Auch hierbei beträgt der Abstand zwischen den einzelnen Verbindungspunkten einer Faser mit dem Grundkörper vorzugsweise zwischen 0,1 und 2,0 cm und insbesondere zwischen 1,0 und 1,5 cm.

Gemäß einer Variante der Erfindung sind die Verbindungspunkte der jeweiligen Fasern voneinander beabstandet, vorzugsweise in einem regelmäßigen Abstand. Dadurch wird in der Sekundärform eine Struktur erzielt, bei welcher sich die einzelnen quer durch die Konusform erstreckenden Elemente gegenseitig abstützen.

Zweckmäßigerweise besteht der Grundkörper aus einem Material mit Formgedächtnis, insbesondere aus Nitinol oder einem Kunststoff mit Formgedächtnis. Dadurch kann sich der Grundkörper beispielsweise beim Verlassen des Implantationskatheters selbständig von der Primärform in die Sekundärform entfalten bzw. aufwickeln.

Nach einer erfindungsgemäßen Variante ist der Grundkörper aus einem drahtartigen Element geformt, wobei der Grundkörper die Form einer Wendel aufweist. Dadurch weist der Grundkörper eine hohe Flexibilität bei gleichzeitiger ausreichender Stabilität auf um in der Sekundärform eine aufgewickelte Konusform anzunehmen.

Zweckmäßigerweise ist in Inneren der des wendelförmigen Grundkörpers ein Innenmandrin angeordnet. Mittels des Innenmandrins lässt sich das medizinische Implantat z.B. auf eine einfach Art und Weise in den die Primärform bringen.

Nach einer erfindungsgemäßen Variante der Erfindung besteht die Konusform aus zwei ineinander gesteckten Trichtern. Dadurch wird die Stabilität des medizinischen Implantats in der Sekundärform verbessert.

In einer besonders bevorzugten Variante der Erfindung ist die wenigstens eine Faser eine thrombogene Faser.

Gemäß einer Variante der Erfindung besteht die wenigstens eine thrombogene Faser aus einer Kunststofffaser, beispielsweise ausgewählt aus der Gruppe enthaltend absorbierende und nicht absorbierende Materialien, natürliche und synthetische Stoffe, insbesondere Polyester, Polyamide, Polypropylen, Polybutylester, expandiertes Polytetrafluorethylen (ePTFE), Polyvinyldifluorethylen (PVDF), Nylon, Leinen, Seide, Katgut.

Nach einer zweckmäßigen Variante der Erfindung weist die aufgewickelte Sekundärform einen ersten konisch zulaufenden Abschnitt, einen sich an dessen Ende mit geringerem Durchmesser anschließenden zylindrischen Abschnitt und einen von diesem auf der Außenseite des ersten konisch zulaufenden Abschnitts in Richtung zu dessen Ende mit größerem Durchmesser sich erstreckenden, um diesen zumindest teilweise herumgewundenen dritten Abschnitt auf.

In einer erfindungsgemäßen Variante weist das medizinische Implantat in der Sekundärform im erweiterten Bereich einen Durchmesser zwischen 2,0 und 20,0 mm auf, vorzugsweise zwischen 8,0 und 16,0 mm. Im spitzzulaufenden Bereich weist das medizinische Implantat in der Sekundärform beispielsweise einen Durchmesser zwischen 1,0 und 10,0 mm auf, vorzugsweise zwischen 6,0 und 8,0 mm.

Der zylindrische Abschnitt einer erfindungsgemäßen Variante weist zum Beispiel einen Durchmesser zwischen 1,0 und 7,0 mm auf, vorzugsweise zwischen 5,0 und 6,5 mm.

Nachfolgend wird die Erfindung anhand der in den Figuren dargestellten Ausführungsbeispiele näher erläutert. Es zeigen:
- Fig. 1: eine perspektivische Ansicht eines erfindungsgemäßen medizinischen Implantats in einer Primärform,
- Fig. 2: eine perspektivische Ansicht des medizinischen Implantats aus Fig. 1 bei einem Übergang von der Primärform in eine Sekundärform,
- Fig. 3: eine perspektivische Ansicht des medizinischen Implantats aus den Figuren 1 und 2 in der Sekundärform,
- Fig.4: eine weitere perspektivische Ansicht des medizinischen Implantats aus Fig. 3 in der Sekundärform,
- Fig. 5: eine schematische Teilansicht einer ersten erfindungsgemäßen Ausführungsform in einer Primärform,
- Fig. 6: eine schematische Teilansicht einer zweiten erfindungsgemäßen Ausführungsform in einer Primärform,
- Fig. 7: eine schematische Teilansicht einer dritten erfindungsgemäßen Ausführungsform in einer Primärform, und
- Fig. 8: eine schematische Teilansicht einer vierten erfindungsgemäßen Ausführungsform in einer Primärform.

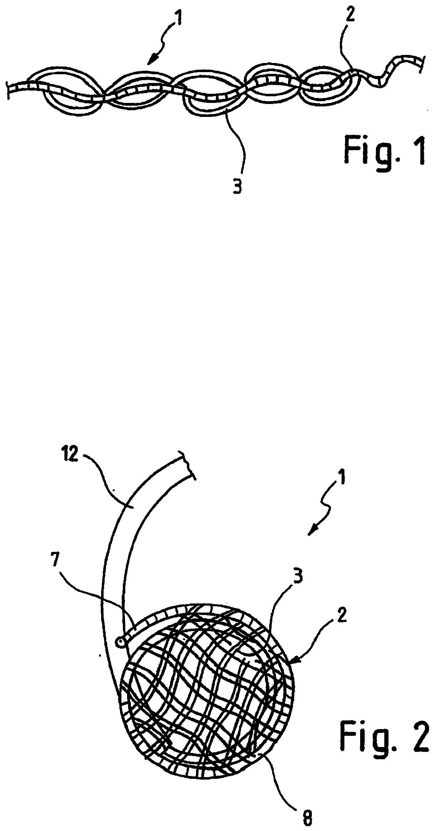

Figur 1 zeigt eine perspektivische Ansicht eines erfindungsgemäßen medizinischen Implantats 1 zum Verschluss einer Defektöffnung, eines Gefäßes, eines Organwegs oder einer anderen Öffnung in einem menschlichen oder tierischen Körper. Das medizinische Implantat 1 aus Figur 1 umfasst einen Grundkörper 2 und wenigstens eine Faser 3, wobei die Faser 3 vorzugsweise thrombogen ist. Auch wenn im Folgenden die Erfindung im Zusammenhang mit wenigstens einer thrombogenen Faser erläutert wird, können grundsätzlich auch andere Arten von Fasern verwendet werden.

Der Grundkörper 2 ist gegen elastische Materialkräfte reversibel von einer Sekundärform in eine Primärform überführbar. In der Primärform weist der Grundkörper 2 eine langgestreckte Form auf und in der Sekundärform ist der Grundkörper 2 zumindest teilweise aufgewickelt und weist eine Konusform 4 auf.

Das erfindungsgemäße medizinische Implantat 1 zeichnet sich dadurch aus, dass die wenigstens eine thrombogene Faser 3 derart mit dem Grundkörper 2 verbunden ist, dass sich die thrombogene Faser 3 in der Sekundärform des Grundkörpers 2 wenigstens einmal, vorzugsweise mehrmals, quer durch die Konusform 4 erstreckt.

Der Erfindung liegt die Erkenntnis zugrunde, dass sich die wenigstens eine thrombogene Faser 3 des medizinischen Implantats 1 im nassen Zustand nicht derart an die Innenwandung der Konusform 4 anlegen kann, dass in dem spitzzulaufenden Bereich der Konusform 4 eine Öffnung verbleibt, wenn sich die wenigstens eine thrombogene Faser 3 quer durch die Konusform 4 erstreckt.

In der Primärform weist der Grundkörper 2 eine langgestreckte Form auf, damit er mittels einer minimal-invasiven Kathetertechnik in den menschlichen oder tierischen Körper implantierbar ist. In dieser Primärform weist der Grundkörper 2 ein großes Verhältnis von Längsausdehnung zu Querausdehnung auf.

Beim Verlassen des zur Implantation verwendeten Katheters 12 nimmt der Grundkörper 2 vorzugsweise selbständig die Sekundärform an, in welcher das aus dem Grundkörper 2 geformte medizinische Implantat 1 die Defektöffnung, das Gefäß, den Organweg oder die andere Körperöffnung in dem menschlichen oder tierischen Körper verschließt. In dieser Sekundärform weist der Grundkörper 2 ein kleineres Verhältnis von Längsausdehnung zu Querausdehnung auf als in der Primärform.

Eine Konusform 4 im Sinne der Erfindung umfasst einen erweiterten Bereich 5 und einen spitzzulaufenden Bereich 6. Die Konusform 4 entspricht somit einer Trichterform. Vorzugsweise ist der erweiterte Bereich 5 der Konusform 4 am distalen Ende 7 des medizinischen Implantats 1 angeordnet und der spitzzulaufende Bereich 6 der Konusform ist am proximalen Ende des medizinischen Implantats 1 angeordnet. Distal im Sinne der Erfindung ist der der Körpermitte zugewandte Bereich 7 und proximal im Sinne der Erfindung ist der der Körpermitte abgewandte Bereich des medizinischen Implantats 1.

Ferner hat die erfindungsgemäße Ausgestaltung des medizinischen Implantats 1 ein verringertes Hämolyserisiko, da die Anzahl der verwendeten thrombogenen Fasern 3 deutlich reduziert wurde. Die losen Enden der thrombogenen Fasern 3 können zu einer Zerstörung von Erythrozyten beitragen, was ein maßgebender Faktor für eine Hämolyse ist.

Die wenigstens eine thrombogene Faser 3 ist an wenigstens zwei Punkten mit dem Grundkörper 2 verbunden. Eine derartige Ausgestaltung führt auf einfache Art und Weise dazu, dass sich die wenigstens eine thrombogene Faser 3 in der Sekundärform des Grundkörpers 2 wenigstens einmal quer durch die Konusform 4 erstreckt. Ferner verläuft die wenigstens eine thrombogene Faser 3 in der Primärform des Grundkörpers 2 nahezu parallel zu dem Grundkörper 2, so dass die wenigstens eine thrombogene Faser 3 nahezu keinen Einfluss auf den Durchmesser des medizinischen Implantats 1 in der Primärform hat. Vorzugsweise steht die wenigstens eine thrombogene Faser 3 in der Primärform des Grundkörpers 2 maximal 0,5 cm von dem Grundkörper 2 ab. Der Durchmesser des medizinischen Implantats 1 in der Primärform ist insbesondere für die minimalinvasive Implantation von Bedeutung, da der Durchmesser des medizinischen Implantats 1 die Größe des zu verwendenden Katheters 12 maßgebend beeinflusst. Vorzugsweise ist die wenigstens eine thrombogene Faser 3 an mehr als zwei Punkten mit dem Grundkörper 2 verbunden. Somit ist die wenigstens eine thrombogene Faser 3 in der Primärform des Grundkörpers 2 wellenförmig an oder um den Grundkörper 2 angeordnet und in der Sekundärform erstrecken sich jeweils die Bereiche der wenigstens einen thrombogenen Faser 3 die nicht mit dem Grundkörper 2 verbunden sind quer durch die Konusform.

In einer besonders bevorzugten Variante der Erfindung sind die wellenförmigen Abschnitte der thrombogenen Faser 3 auf einer Seite des Grundkörpers angeordnet.

Nach einer weiteren Variante der Erfindung umfasst der Grundkörper 2 des medizinischen Implantats 1 wenigstens zwei thrombogene Fasern 3, welche jeweils an wenigstens zwei Punkten mit dem Grundkörper 2 verbunden sind.

Zweckmäßigerweise besteht der Grundkörper 2 des medizinischen Implantats 1 aus einem Formgedächtnismaterial, insbesondere aus Nitinol oder einem Kunststoff mit Formgedächtnis. Durch die Verwendung eines Formgedächtnismaterials wird erreicht, dass sich das medizinische Implantat 1, insbesondere dessen Grundkörper 2, in einer vorbestimmten Art und Weise von der Primärform in die Sekundärform umwandelt bzw. entfaltet. Dies ist insbesondere für eine Implantation mittels einer minimal-invasiven Kathetertechnik vorteilhaft.

Der Grundkörper 2 des medizinischen Implantats 1 aus Figur 1 ist aus einem drahtartigen Element 8 geformt und weist dir Form einer Wendel auf. Das drahtartige Element 8 des Grundkörpers 2 ist also zu einer Wendel geformt. Ein als Wendel ausgebildeter Grundkörper 2 weist eine hohe Flexibilität in Längsrichtung wie auch in Radialrichtung auf, bei einer gleichzeitigen hinreichenden Stabilität um die Defektöffnung, das Gefäß, den Organweg oder die andere Öffnung im menschlichen oder tierischen Körper zu verschließen.

Die wenigstens eine thrombogene Faser 3 besteht in dem Ausführungsbeispiel gemäß Figur 1 aus einer Kunststofffaser, beispielsweise ausgewählt aus der Gruppe enthaltend absorbierende und nicht absorbierende Materialien, natürliche und synthetische Stoffe, insbesondere Polyester, Polyamide, Polypropylen, Polybutylester, expandiertes Polytetrafluorethylen (ePTFE), Polyvinyldifluorethylen (PVDF), Nylon, Leinen, Seide, Katgut.

Figur 2 zeigt eine perspektivische Ansicht des medizinischen Implantats 1 aus Fig. 1 bei einem Übergang von der Primärform in eine Sekundärform. Dazu wickelt sich der Grundkörper 2 des medizinischen Implantats 1 beim Verlassen des zur Implantation verwendeten Katheters 12 derart auf, dass der Grundkörper 2 eine Konusform 4 bildet. Die Konusform 4 umfasst einen erweiterten Bereich 5 und einen spitzzulaufenden Bereich 6. Beim Verlassen des Katheters 12 wickelt sich der Grundkörper 2 von seinem distalen Ende 7 her auf und bildet zunächst den erweiterten Bereich 5 der Konusform 4. Dieser Zustand ist in Figur 2 dargestellt.

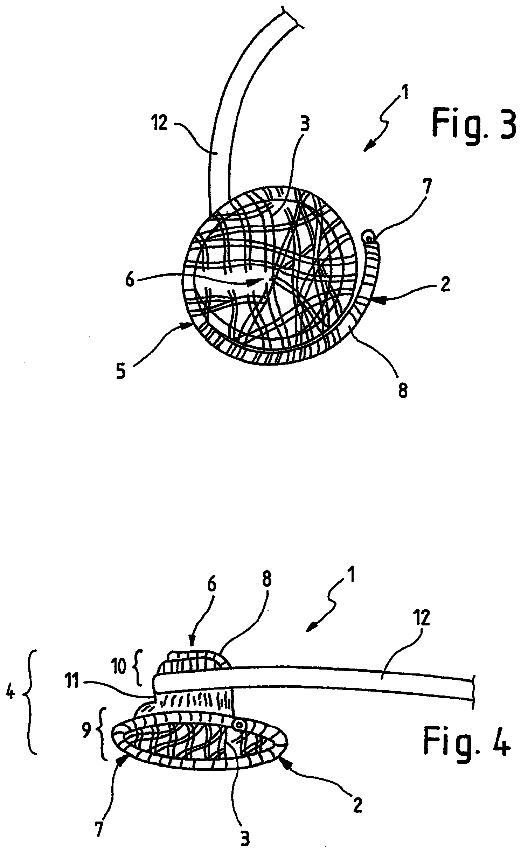

Im weiteren Verlauf der Implantation wird der Grundkörper 2 weiter aus dem Katheter 12 geschoben und der Grundkörper wickelt bzw. faltet sich weiter von der Primärform in die Sekundärform, also von einer langgestreckten Form in die Konusform 4. Figur 3 zeigt eine perspektivische Ansicht des medizinischen Implantats 1 aus den Figuren 1 und 2 in der Sekundärform, also nachdem auch das proximale Ende des Grundkörpers 2 den Katheter 12 verlassen hat. Die Ansicht aus Figur 3 zeigt das medizinische Implantat 1 in einer Draufsicht auf den erweiterten Bereich 5. Der spitzzulaufende Bereich 6 befindet sich demnach mittig in dem dargestellten medizinischen Implantat 1. Der Figur 3 lässt sich insbesondere entnehmen, dass die wenigstens eine thrombogene Faser 3 in der Konusform 4 eine netzartige Struktur bildet.

Figur 4 zeigt eine weitere perspektivische Ansicht des medizinischen Implantats 1 aus Fig. 3 in der Sekundärform. Diese Ansicht entspricht einer Seitenansicht des medizinischen Implantats 1. Figur 3 lässt sich entnehmen, dass die aufgewickelte Sekundärform einen ersten konisch zulaufenden Abschnitt 9, einen sich an dessen Ende mit geringerem Durchmesser anschließenden zylindrischen Abschnitt 11 und einen von diesem auf der Außenseite des ersten konisch zulaufenden Abschnitts 9 in Richtung zu dessen Ende mit größerem Durchmesser 7 sich erstreckenden, um diesen zumindest teilweise herumgewundenen dritten Abschnitt 10 aufweist. Die Konusform 4 besteht folglich aus zwei ineinander gesteckten Trichtern.

Das medizinische Implantat 1 aus den Figuren 1 bis 4 weist in der Sekundärform in dem erweiterten Bereich 5 einen Durchmesser zwischen 2,0 und 20,0 mm auf, vorzugsweise zwischen 8,0 und 16, 0 mm. In dem spitzzulaufenden Bereich 6 des medizinischen Implantats 1 weist das medizinische Implantat 1 in der Sekundärform einen Durchmesser zwischen 1,0 und 10,0 mm, vorzugsweise zwischen 6,0 und 8,0 mm. Der zylindrische Abschnitt 11 hat einen Durchmesser zwischen 1,0 und 7,0 mm, vorzugsweise zwischen 5,0 und 6,5 mm. Die Abmessungen des medizinischen Implantats 1 sind an die zu verschließende Defektöffnung, das Gefäß, den Organweg oder der anderen Öffnung angepasst.

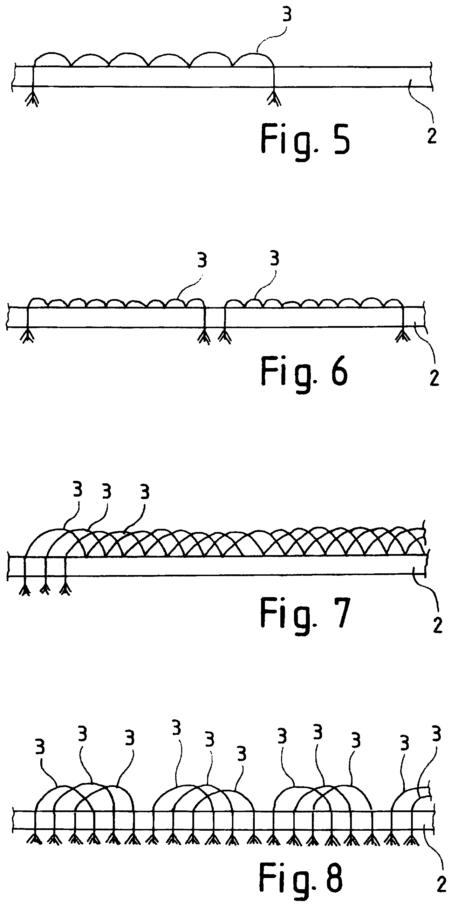

Figur 5 zeigt eine schematische Teilansicht einer ersten erfindungsgemäßen Ausführungsform in einer Primärform, Figur 6 eine schematische Teilansicht einer zweiten erfindungsgemäßen Ausführungsform in einer Primärform, Figur 7 eine schematische Teilansicht einer dritten erfindungsgemäßen Ausführungsform in einer Primärform, und Figur 8 eine schematische Teilansicht einer vierten erfindungsgemäßen Ausführungsform in einer Primärform. Die Teilansichten aus den Figuren 5 bis 8 zeigen jeweils einen Abschnitt eines Grundkörpers 2 eines erfindungsgemäßen medizinischen Implantats 1 und daran angeordnet wenigstens eine thrombogene Faser 3.

Gemäß der Figur 5 ist an dem Grundkörper 2 eine thrombogene Faser 3 angeordnet. Die thrombogene Faser 3 besteht aus einer Vielzahl von einzelnen Filamenten, wodurch die Flexibilität der thrombogenen Faser 3 verbessert wird. An den Endpunkten ist die thrombogene Faser derart an dem Grundkörper 2 befestigt, dass die thrombogene Faser durch den Grundkörpers 2 hindurchläuft, so dass die einzelnen Filamente der thrombogenen Faser 3 aus dem Grundkörper herausragen. Alternativ könnte die thrombogene Faser 3 auch innerhalb der Grundkörpers 2 befestigt sein. Ferner ist die thrombogene Faser 3 an fünf weiteren Stellen mit dem Grundkörper 2 verbunden. In der Ausgestaltung gemäß Figur 5 ist die thrombogene Faser 3 an diesen Zwischenverbindungsstellen innerhalb des Grundkörpers 2 mit dem Grundkörper 2 verbunden. Alternativ können diese Verbindungsstellen auch wie die Endverbindungsstellen ausgebildet werden, so dass die thrombogene Faser 3 an diesen Zwischenverbindungsstellen ebenfalls durch den Grundkörper 2 hindurchläuft. Zweckmäßigerweise ist die thrombogene Faser 3 zumindest im Bereich des distalen Endes 7 des Grundkörpers 2 angeordnet.

In der Ausführungsform gemäß Figur 6 sind zwei thrombogene Fasern 3 an dem Grundkörper 2 angeordnet. Diese thrombogenen Fasern 3 sind jeweils an ihren Enden mit dem Grundkörper 2 verbunden, so dass die thrombogene Faser 3 durch den Grundkörper 2 hindurchläuft, und an acht Zwischenverbindungspunkten mit dem inneren des Grundkörpers 2 verbunden. Die Verbindungsart zwischen thrombogener Faser 3 und Grundkörper 2 ist jedoch grundsätzlich frei wählbar. Die beiden thrombogenen Fasern 3 der Ausführungsform aus Figur 6 sind nebeneinander angeordnet und überlappen sich nicht.

Nach der Ausführungsform aus Figur 7 sind drei thrombogene Faser 3 an dem Grundkörper 2 angeordnet. Wie bezüglich der Ausführungsformen aus den Figuren 5 und 6 beschrieben sind die thrombogenen Fasern 3 an deren Enden und an einer Mehrzahl von Zwischenpunkten mit dem Grundkörper 2 verbunden. Die Ausführungsform gemäß Figur 7 unterscheidet sich von den vorherigen Ausführungsformen gemäß der Figuren 5 und 6 dadurch, dass die einzelnen thrombogenen Fasern 3 untereinander verschränkt an dem Grundkörper 2 angeordnet sind.

Gemäß der Ausführungsform aus Figur 8 sind jeweils drei thrombogene Fasern 3 untereinander verschränkt mit dem Grundkörper 2 verbunden, wobei die thrombogenen Fasern 3 nur an ihren Enden mit dem Grundkörper 2 verbunden sind. Nachfolgend ist eine neue Anordnung bestehend aus drei untereinander verschränkten thrombogenen Fasern 3 an dem Grundkörper 2 angeordnet.

In den Ausführungsformen gemäß den Figuren 5 bis 8 sind die Verbindungspunkte der jeweiligen thrombogenen Fasern 3 voneinander beabstandet, vorzugsweise in einem regelmäßigen Abstand.

Die einzelnen Ausführungsformen gemäß der Figuren 5 bis 8 können auch untereinander kombiniert werden. Insbesondere ist die Anzahl der thrombogenen Faser 3, die Anzahl der Verbindungsstellen zwischen thrombogener Faser 3 und Grundkörper 2, die Ausgestaltung der Verbindung zwischen thrombogener Faser 3 und Grundkörper, die Art und Ausgestaltung einer Verschränkung von thrombogenen Fasern 3 und so weiter durch den Fachmann bei der Umsetzung der vorliegenden Erfindung im Rahmen der Ansprüche frei wählbar.

### Bezugszeichenliste

- 1: medizinisches Implantat
- 2: Grundkörper
- 3: thrombogene Faser
- 4: Konusform
- 5: erweiterter Bereich
- 6: spitzzulaufender Bereich
- 7: distales Ende
- 8: drahtartiges Element
- 9: erster konischer Abschnitt
- 10: dritter Abschnitt
- 11: zylindrischer Abschnitt
- 12: Katheter

## Patentansprüche

1. Medizinisches Implantat (1) zum Verschluss einer Defektöffnung, eines Gefäßes, eines Organwegs oder einer anderen Öffnung in einem menschlichen oder tierischen Körper,
mit einem Grundkörper (2) und wenigstens einer Faser (3),
wobei der Grundkörper (2) gegen elastische Materialkräfte reversibel von einer Sekundärform in eine Primärform überführbar ist,
wobei der Grundkörper (2) in der Primärform eine langgestreckte Form aufweist und in der Sekundärform zumindest teilweise aufgewickelt ist und eine Konusform (4) aufweist,
**dadurch gekennzeichnet, dass**
die wenigstens eine Faser (3) an wenigstens zwei Punkten mit dem Grundkörper (2) verbunden ist, so dass sich die Faser (3) in der Sekundärform des Grundkörpers (2) wenigstens einmal, vorzugsweise mehrmals, quer durch die Konusform (4) erstreckt und in der Konusform (4) eine netzartige Struktur bildet.

2. Medizinisches Implantat (1) nach Anspruch 1, **dadurch gekennzeichnet, dass** sich die wenigstens eine Faser (3) wenigstens einmal durch den Mittelpunkt der Konusform (4) erstreckt.

3. Medizinisches Implantat (1) nach einem der Ansprüche 1 bis 2, **dadurch gekennzeichnet, dass** die wenigstens eine Faser (3) an mehr als zwei Punkten mit dem Grundkörper (2) verbunden ist.

4. Medizinisches Implantat (1) nach Anspruch 3, **dadurch gekennzeichnet, dass** wenigstens zwei Fasern (3) jeweils an wenigstens zwei Punkten mit dem Grundkörper (2) verbunden sind.

5. Medizinisches Implantat (1) nach Anspruch 4, **dadurch gekennzeichnet, dass** die Verbindungspunkte der jeweiligen Fasern (3) voneinander beabstandet sind, vorzugsweise in einem regelmäßigen Abstand.

6. Medizinisches Implantat (1) nach einem der Ansprüche 1 bis 5, **dadurch gekennzeichnet, dass** der Grundkörper (2) aus einem Material mit Formgedächtnis besteht, insbesondere aus Nitinol oder einem Kunststoff mit Formgedächtnis.

7. Medizinisches Implantat (1) nach einem der Ansprüche 1 bis 6, **dadurch gekennzeichnet, dass** der Grundkörper (2) aus einem drahtartigen Element (9) geformt ist, wobei der Grundkörper (2) die Form einer Wendel aufweist.

8. Medizinisches Implantat (1) nach einem der Ansprüche 1 bis 7, **dadurch gekennzeichnet, dass** die Konusform (4) aus zwei ineinander gesteckten Trichtern besteht.

9. Medizinisches Implantat (1) nach einem der Ansprüche 1 bis 8, **dadurch gekennzeichnet, dass** die wenigstens eine Faser eine thrombogene Faser ist.

10. Medizinisches Implantat (1) nach Anspruch 9, **dadurch gekennzeichnet, dass** die wenigstens eine thrombogene Faser (3) aus einer Kunststofffaser besteht, beispielsweise ausgewählt aus der Gruppe enthaltend absorbierende und nicht absorbierende Materialien, natürliche und synthetische Stoffe, insbesondere Polyester, Polyamide, Polypropylen, Polybutylester, expandiertes Polytetrafluorethylen (ePTFE), Polyvinyldifluorethylen (PVDF), Nylon, Leinen, Seide, Katgut.

11. Medizinisches Implantat (1) nach einem der Ansprüche 1 bis 10, **dadurch gekennzeichnet, dass** die aufgewickelte Sekundärform einen ersten konisch zulaufenden Abschnitt (9), einen sich an dessen Ende mit geringerem Durchmesser anschließenden zylindrischen Abschnitt (11) und einen von diesem auf der Außenseite des ersten konisch zulaufenden Abschnitts (9) in Richtung zu dessen Ende mit größerem Durchmesser sich erstreckenden, um diesen zumindest teilweise herumgewundenen dritten Abschnitt (10) aufweist.

12. Medizinisches Implantat (1) nach einem der Ansprüche 1 bis 11, **dadurch gekennzeichnet, dass** das medizinische Implantat (1) in der Sekundärform im erweiterten Bereich (5) einen Durchmesser zwischen 2,0 und 20,0 mm aufweist, vorzugsweise zwischen 8,0 und 16,0 mm.

13. Medizinisches Implantat (1) nach einem der Ansprüche 1 bis 12, **dadurch gekennzeichnet, dass** das medizinische Implantat (1) in der Sekundärform im spitzzulaufenden Bereich (6) einen Durchmesser zwischen 1,0 und 10,0 mm aufweist, vorzugsweise zwischen 6,0 und 8,0 mm.

14. Medizinisches Implantat (1) nach Anspruch 11, **dadurch gekennzeichnet, dass** der zylindrische Abschnitt (11) einen Durchmesser zwischen 1,0 und 7,0 mm aufweist, vorzugsweise zwischen 5,0 und 6,5 mm.

## Claims

1. A medical implant (1) for closure of a defect aperture, a vessel, an organ path or another aperture in a human or animal body, comprising:
a base body (2) and at least one fibre (3),
wherein the base body (2) is reversibly transformable against elastic material forces from a secondary shape into a primary shape,
wherein in the primary shape the base body (2) has an elongated shape and in the secondary shape is at least partially coiled and comprises a cone shape (4),
wherein the at least one fibre (3) is at least at two points connected to the base body (2) such that the at least one fibre (3) in the secondary shape of the base body (2) extends at least once, preferably several times, transversely through the cone shape (4) and forming a net-like structure in the cone shape (4).

2. Medical implant (1) according to claim 1, wherein the at least one fibre (3) extends at least once through the centre of the cone shape (4).

3. Medical implant (1) according to any of claims1 to 2, wherein the at least one fibre (3) is connected to the base body (2) at more than two connecting points.

4. Medical implant (1) according to claim 3, wherein at least two fibres (3) are connected to the base body (2) at at least two connecting points each.

5. Medical implant (1) according to claim 4, wherein the connecting points of the respective fibres (3) are spaced from one another, preferably at a regular distance.

6. Medical implant (1) according to any of claims 1 to 5, wherein the base body (2) is formed of a material with a shape memory, in particular of nitinol or plastic with a shape memory.

7. Medical implant (1) according to any of claims 1 to 6, wherein the base body (2) is formed from a wire-like element (9), wherein the base body (2) has the form of a helix.

8. Medical implant (1) according to any of claims 1 to 7, wherein the cone shape (4) consists of two funnels inserted one inside the other.

9. Medical implant (1) according to any of claims 1 to 8, wherein the at least one fibre is a thrombogenic fibre.

10. Medical implant (1) according to claim 9, wherein the at least one thrombogenic fibre (3) consists of a plastic fibre, for example selected from the group comprising absorbent and non-absorbent materials, natural and synthetic materials, in particular polyester, polyamide, polypropylene, polybutyl ester, expanded polytetrafluoroethylene (ePTFE), polyvinyldifluoroethylene (PVDF), nylon, linen, silk or catgut.

11. Medical implant (1) according to any of claims 1 to 10, wherein the coiled secondary shape comprises a first conical tapered region (9), a cylindrical region (11) adjoining the end thereof with a smaller diameter and a third section (10) extending from the cylindrical section (11) on the outside of the first conically tapering section (9) in the direction of the end thereof with a larger diameter and wound at least partly around the third section (10).

12. Medical implant (1) according to any of claims 1 to 11, wherein the medical implant (1) in the secondary shape has a diameter between 2,0 and 20,0 mm, preferably between 8,0 and 16,0 mm, in the extended region (5).

13. Medical implant (1) according to any of claims 1 to 12, wherein the medical implant (1) in the secondary shape has a diameter between 1,0 and 10,0 mm, preferably between 6,0 and 8,0 mm, in the tapered region (6).

14. Medical implant (1) according to claim 11, wherein the cylindrical region (11) has a diameter between 1.0 and 7.0 mm, preferably between 5,0 and 6,5 mm.

## Revendications

1. Implant médical (1) destiné à la fermeture d'une ouverture défectueuse, d'un vaisseau, d'un trajet d'organe ou d'une autre ouverture dans un corps humain ou animal, comprenant :
un corps de base (2), et au moins une fibre (3),
dans lequel le corps de base (2) est transformable de manière réversible contre des forces de matériau élastique d'une forme secondaire en une forme primaire,
dans lequel dans la forme primaire, le corps de base (2) présente une forme allongée et dans la forme secondaire, il est au moins partiellement enroulé et comprend une forme conique (4),
dans lequel l'au moins une fibre (3) est au moins connectée au niveau de deux points au corps de base (2) de sorte que l'au moins une fibre (3) dans la forme secondaire du corps de base (2) s'étend au moins une fois, de préférence plusieurs fois, transversalement à travers la forme conique (4) et formant une structure de type filet dans la forme conique (4).

2. Implant médical (1) selon la revendication 1, dans lequel l'au moins une fibre (3) s'étend au moins une fois à travers le centre de la forme conique (4).

3. Implant médical (1) selon l'une quelconque des revendications 1 à 2, dans lequel l'au moins une fibre (3) est connectée au corps de base (2) au niveau de plus de deux points de connexion.

4. Implant médical (1) selon la revendication 3, dans lequel au moins deux fibres (3) sont connectées au corps de base (2) au niveau d'au moins deux points de connexion chacune.

5. Implant médical (1) selon la revendication 4, dans lequel les points de connexion des fibres respectives (3) sont espacés les uns des autres, de préférence à une distance régulière.

6. Implant médical (1) selon l'une quelconque des revendications 1 à 5, dans lequel le corps de base (2) est formé d'un matériau à mémoire de forme, en particulier de nitinol ou de plastique à mémoire de forme.

7. Implant médical (1) selon l'une quelconque des revendications 1 à 6, dans lequel le corps de base (2) est formé d'un élément filaire (9), le corps de base (2) ayant la forme d'une hélice.

8. Implant médical (1) selon l'une quelconque des revendications 1 à 7, dans lequel la forme conique (4) est constituée de deux entonnoirs insérés l'un à l'intérieur de l'autre.

9. Implant médical (1) selon l'une quelconque des revendications 1 à 8, dans lequel l'au moins une fibre est une fibre thrombogénique.

10. Implant médical (1) selon la revendication 9, dans lequel l'au moins une fibre thrombogénique (3) est constituée d'une fibre plastique, par exemple sélectionnée à partir du groupe comprenant des matériaux absorbants et non absorbants, des matériaux naturels et synthétiques, en particulier de polyester, de polyamide, de polypropylène, d'ester de polybutyle, de polytétrafluoroéthylène expansé (ePTFE), de polyvinyldifluoroéthylène (PVDF), de nylon, de lin, de soie ou de catgut.

11. Implant médical (1) selon l'une quelconque des revendications 1 à 10, dans lequel la forme secondaire enroulée comprend une première région conique effilée (9), une région cylindrique (11) adjacente à l'extrémité de celle-ci ayant un diamètre inférieur et une troisième section (10) s'étendant de la section cylindrique (11) sur l'extérieur de la première section effilée de manière conique (9) dans la direction de l'extrémité de celle-ci ayant un diamètre supérieur et enroulée au moins partiellement autour de la troisième section (10).

12. Implant médical (1) selon l'une quelconque des revendications 1 à 11, dans lequel l'implant médical (1) dans la forme secondaire présente un diamètre situé entre 2,0 et 20,0 mm, de préférence entre 8,0 et 16,0 mm, dans la région étendue (5).

13. Implant médical (1) selon l'une quelconque des revendications 1 à 12, dans lequel l'implant médical (1) dans la forme secondaire présente un diamètre situé entre 1,0 et 10,0 mm, de préférence entre 6,0 et 8,0 mm, dans la région effilée (6).

14. Implant médical (1) selon la revendication 11, dans lequel la région cylindrique (11) présente un diamètre situé entre 1,0 et 7,0 mm, de préférence entre 5,0 et 6,5 mm.
